# EUROPÄISCHE PATENTSCHRIFT

(11) **EP 0 757 546 B1**
(45) Veröffentlichungstag und Bekanntmachung des Hinweises auf die Patenterteilung: **23.09.1998**
(21) Anmeldenummer: 95919354.1
(22) Anmeldetag: 28.04.1995
(51) Int. Cl.: A61B 17/39

(54) **ELEKTROCHIRURGISCHES INSTRUMENT ZUR THERAPIE VON VARIZEN**
ELECTROSURGICAL INSTRUMENT FOR THERAPEUTIC TREATMENT OF VARICES
INSTRUMENT ELECTROCHIRURGICAL POUR TRAITER LES VARICES

(30) Priorität: 28.04.1994 DE 4414807
(43) Veröffentlichungstag der Anmeldung: 12.02.1997
(73) Patentinhaber: Willmen, Hans Rainer Prof. Dr. med., D-41515 Grevenbroich (DE)
(72) Erfinder: Willmen, Hans Rainer Prof. Dr. med., D-41515 Grevenbroich (DE)
(74) Vertreter: Behrendt, Arne, Dipl.-Ing.
(86) Internationale Anmeldenummer: EP9501614
(87) Internationale Veröffentlichungsnummer: WO9529644

(56) Entgegenhaltungen:
- US-A- 1 814 791
- US-A- 1 916 722
- US-A- 1 943 543
- US-A- 2 611 365
- US-A- 3 301 258
- US-A- 3 651 812

## Beschreibung

Die Erfindung betrifft ein elektrochirurgisches Instrument zur Therapie von Varizen, insbesondere Besenreiser-Varizen.

Unter Varizen versteht man entlang der Körperoberfläche verlaufende Venen, die meist sackartig oder knotenförmig pathologisch erweitert sind. Durch stehende Berufe, Übergewicht, enge Kleidung oder Schwangerschaft entwickeln sich aus einer angeborenen Bindegewebsschwäche besonders häufig sogenannte Besenreiser-Varizen der Beinvenen. Angesichts der zivilisationsbedingten Zunahme dieser begünstigenden Faktoren leiden immer mehr Menschen an den Folgen der dadurch ausgelösten primären Varikosis. Im fortgeschrittenen Stadium sind Varizen mitunter sehr schmerzhaft und führen nicht selten zu Arbeitsunfähigkeit. Deswegen ist eine möglichst frühzeitige Therapie von Besenreisern und kleinen Varizen angezeigt.

Nach dem Stand der Technik ist die sogenannte Sklerosierung zur Behandlung von Besenreisern üblich. Dabei wird nach einer Luft-Vorinjektion ein Verödungsmittel in den entsprechenden Venenabschnitt injiziert. Nachfolgend schließt sich eine Kompressionsbehandlung, z. B. durch Kompressionsverbände oder -strümpfe an.

Die vorgenannte Methode hat den Vorteil, daß insbesondere bei der Behandlung von Besenreisern ein operativer Eingriff nicht notwendig ist. Ansonsten führen bekannte Verödungsverfahren jedoch zu unbefriedigenden Ergebnissen: Die medikamentöse Sklerosierung führt oftmals zu mehr oder weniger ausgedehnten, fleckigen Verfärbungen der Haut, was nicht nur als kosmetisch störend empfunden wird, sondern auch eine psychische Belastung des Patienten darstellt. Weiterhin ist die Therapie relativ langwierig, weil mehrere, im Abstand von mehreren Tagen aufeinanderfolgende Behandlungen erforderlich sind. Außerdem ist die Rezidivquote bei der Sklerosierung relativ hoch.

Es werden auch Versuche unternommen, Besenreiser-Varizen durch Bestrahlung mit Laserlicht zu therapieren. Diese Verfahren sind bisher jedoch insuffizient. Es sind damit lediglich geringfügige kosmetische Korrekturen ermöglicht worden. Für eine umfassende Besenreiser- oder Varizenbehandlung scheint die Laser-Therapie nach dem derzeitigen Erkenntnisstand wenig erfolgversprechend.

Aus dem US-Patent Nr. 3 301 258 ist ein Verfahren zur Behandlung von Varizen bekannt, welches auf der Zerstörung des erkrankten Venenabschnittes mittels elektrischen Stroms (Diathermiestrom) beruht. Das in der US-Schrift ebenfalls offenbarte Instrument zur Durchführung des Verfahrens weist zwei voneinander elektrisch isolierte Elektroden auf, die am distalen Ende einer schmalen zylindrischen Sonde angeordnet sind, sowie Mittel zur Erzeugung und Übertragung von elektrischem Strom auf die vorgenannten Elektroden.

Zur Behandlung der Varize wird die Sonde durch einen Einschnitt in die Haut des Patienten eingeführt und subkutan so weit geführt, bis die am distalen Ende der Sonde vorgesehenen Elektroden sich an der zu behandelnden Stelle befinden. Anschließend läßt man einen elektrischen Strom durch die Elektroden fließen; die dabei erzeugte Wärme führt zu der Zerstörung des benachbarten Venenabschnittes. Das chirurgische Instrument zur Behandlung von Varizen gemäß dem vorgenannten US-Patent ist für die ambulante Therapie jedoch nicht geeignet, weil der damit verbundene operative Eingriff einen Krankenhausaufenthalt erforderlich macht und zu einer erheblichen Belastung des Patienten führt.

Aus der US-Druckschrift 1 943 543 ist ein chirurgisches Instrument zur Verwendung bei der Diathermie und ähnlichen medizinischen und chirurgischen Behandlungen bekannt, welches aus einem Griff, einer Isolierhülse und zwei an den Griff befestigten Elektroden besteht. Die beiden Elektroden sind von der Isolierhülse umgeben und können durch Verschiebung der Hülse frei gelassen werden. Beim Einführen der Elektroden in das zu behandelnde Gewebe verbleibt die Isolierhülse über der Gewebeoberfläche, so daß der in das Gewebe hineinragende Elektrodenabschnitt unisoliert bleibt. Das vorgenannte chirurgische Instrument ist zur Therapie von Varizen deshalb nicht geeignet, weil aufgrund der mangelnden Isolierung der Elektroden Strom entlang der Hautoberfläche fließen kann, was zur Entstehung kosmetisch entstellender Strommarken führt.

Daraus ergibt sich die Aufgabe der Erfindung, dem behandelnden Therapeuten ein chirurgisches Instrument an die Hand zu geben, welches eine schnell und sicher durchführbare, den Patienten möglichst wenig belastende Therapie von Besenreiser-Varizen und dergleichen ermöglicht, bei der möglichst geringe Nebenwirkungen auftreten. Des weiteren wird eine möglichst geringe Rezidivquote angestrebt.

Zur Lösung dieser Aufgabe schlägt die Erfindung ausgehend von dem chirurgischen Instrument zur Behandlung von Varizen gemäß dem US-Patent Nr. 3 301 258 vor, daß das Instrument zwei gabelartig angeordnete, vorstehende Nadelelektroden aufweist, die an unterschiedliche Pole einer elektrischen Stromquelle anschließbar sind und die jeweils teilweise mit einer dünnen elektrischen Isolierschicht umgeben sind, welche den vorderen Spitzenbereich der Nadelelektroden frei läßt, wobei der Übergang vom Spitzenbereich zum isolierten Bereich im wesentlichen absatzlos ist.

Die beiden Nadelelektroden sind bei der Erfindung so nebeneinander angeordnet, daß sie eine zweizinkige Gabel bilden. Dabei sind die beiden Nadelelektroden mit einer dünnen elektrischen Isolierschicht versehen, die kurz vor der metallisch blanken Spitze endet. Die Isolierschicht wird so dünn gewählt, daß beim Übergang zur unisolierten Spitze nahezu kein vorspringender Absatz entsteht. Alternativ kann die Nadelelektrode in ihrem hinteren Bereich derart ausgeformt sein, daß die Isolierung versenkt ist, wodurch der isolierte Schaft und die Spitze der Nadelelektrode denselben Querschnitt aufweisen, d.h. der isolierte Bereich nahezu übergangslos an die unisolierte Spitze anschließt. Die beiden Nadelelektroden sind gegeneinander elektrisch isoliert und über ein Anschlußkabel an eine elektrische Stromquelle anschließbar. In der Regel wird als Stromquelle ein HF-(Hochfrequenz)-Versorgungsteil eingesetzt, wie es aus der Elektrochirurgie beispielsweise zur Erwärmung von Gewebe bekannt ist.

Der besondere Vorteil der Erfindung ergibt sich aus der gabelartigen Anordnung der Nadelelektroden zusammen mit deren teilweiser Isolierung. Die erfindungsgemäß gebildete, bipolare Koagulationsgabel wird vom Operateur einfach durch die Hautoberfläche des Patienten in eine Besenreiser-Varize eingestochen, wobei die unisolierten Spitzen der beiden Nadelelektroden hintereinander im Querschnitt einer pathologisch erweiterten Vene angeordnet werden. Durch das Anlegen einer HF-Spannung an die beiden Nadelelektroden fließt ein hochfrequenter elektrischer Strom zwischen den unisolierten Nadelspitzen entlang der Besenreiser-Varize, in die die Koagulationsgabel eingestochen ist. Dadurch wird erreicht, daß lokal eng begrenzt nur im Bereich des Besenreisers zwischen den Nadelelektroden eine Elektro-Koagulation durchgeführt wird.

Je nach dem Abstand der Nadelelektroden voneinander läßt sich somit eine lokalisierte Koagulation von Besenreiser-Varizen gezielt und punktgenau durchführen. Dabei stellt die Elektro-Koagulation ein wirksames Verfahren dar, Besenreiser schon bei kurzer Behandlungsdauer vollständig zu veröden, so daß eine niedrige Rezidivquote, d.h. eine erneute Bildung von Varizen, erreicht wird.

Die Behandlung ist für den Therapeuten einfach und sicher durchführbar. Dabei wird der Patient selbst nur in geringem Maße belastet, weil die Nadelelektroden hinreichend dünn ausgeführt werden können und die Einstichtiefe in der Regel gering ist. Der übergangslose Anschluß der metallisch blanken Nadelspitzen an den isolierten Schaft sorgt dafür, daß das Einstechen der Koagulationsgabel völlig problemlos ist.

Bei der gezielten und lokalisierten Koagulation ist es weiterhin vorteilhaft, daß benachbartes Körpergewebe und Nervenstränge praktisch nicht durch Erwärmung geschädigt werden. Dadurch, daß die Nadelelektroden in ihrem Verlauf mit einer Isolierung versehen sind, fließt der elektrische HF-Strom lokalisiert subkutan. Dabei fließt kein Strom entlang der Kautoberfläche, wodurch keine kosmetisch entstellenden Strommarken auftreten.

Die Elektroden und die Isolierung werden bevorzugt aus Materialien gefertigt, welche die Einhaltung der Sicherheits- und Schutzbestimmungen bei aufgeschaltetem elektrischen Strom gewährleisten. Es ist besonders zweckmäßig, daß diese Materialien wärmestabil sind, so daß sie thermisch sterilisierbar sind.

Eine vorteilhafte Weiterbildung der Erfindung sieht vor, daß die Nadelelektroden am hinteren Ende axial in Isolierhülsen gehaltert sind, die einen deutlich größeren Querschnitt aufweisen als die Nadelelektroden. Durch diese Ausgestaltung wird in vorteilhafter Weise die Verwendung einer erfindungsgemäßen Koagulationsgabel weiter vereinfacht und abgesichert. Dabei entsteht nämlich beim Übergang von den Nadelelektroden zu den Isolierhülsen ein Absatz, der beim Einstechen der Koagulationsgabel als Anschlag dient. Ragen beispielsweise die Nadelelektroden nur wenige Millimeter aus den Isolierhülsen nach vorne vor, so ist es unmöglich, daß die Nadelelektroden versehentlich zu tief in die Haut des Patienten eingestochen werden. Wird die Länge der Nadelelektroden selbst der typischen Tiefe von Besenreiser-Varizen angepaßt, in der diese unter der Hautoberfläche entlanglaufen, ist die Therapie besonders einfach, weil die Koagulationsgabel lediglich bis zu den Isolierhülsen eingestochen werden muß.

Eine besonders vorteilhafte Ausgestaltung sieht weiterhin vor, daß die Isolierhülsen die Schenkel eines in etwa hosenförmigen Elektrodenkopfes bilden, an dem schenkelseitig nach vorne gerichtet die Nadelelektroden befestigt sind und der nach hinten mit einem griffelförmigen Griffstück verbunden ist. Der Vorteil der in etwa hosenförmigen Ausgestaltung des Elektrodenkopfes ist, daß der Therapeut jederzeit freie Sicht auf das Anwendungsgebiet hat. Der hinten an dem Elektrodenkopf angebrachte Handgriff, der etwa griffelförmig ausgebildet ist, sorgt dafür, daß das Instrument besonders gut handhabbar ist. Es kann dann nämlich wie ein Gabelbesteck oder ein Schreibstift bequem mit einer Hand geführt und angesetzt werden.

Eine bevorzugte Weiterbildung der Erfindung sieht vor, daß der Elektrodenkopf und das Griffstück korrespondierende elektrische und mechanische Verbindungselemente aufweisen, die lösbar miteinander verbindbar sind. In dieser Ausführungsform weist das Griffstück beispielsweise einen elektrischen Steckverbinder auf, der mit dem elektrischen Zuleitungskabel verbunden ist und in den der Elektrodenkopf einsteckbar ist. Damit wird erreicht, daß der Elektrodenkopf schnell und problemlos austauschbar ist. Dies ist besonders dann wichtig, wenn beispielsweise Elektrodenköpfe mit unterschiedlich langen Nadelelektroden und/oder unterschiedlichen Abständen der Nadelelektroden voneinander eingesetzt werden sollen. Durch Austausch des Elektrodenkopfes ist das Instrument außerdem sofort wieder einsatzbereit, so daß Patienten ohne Wartezeiten nacheinander therapiert werden können. Währenddessen können die ausgetauschen Elektrodenköpfe wiederum sterilisiert werden.

Zur Vermeidung von elektrischen Verlusten ist es besonders zweckmäßig, daß die elektrischen und mechanischen Verbindungselemente koaxiale Steckverbinder sind. Einerseits wird damit ein verlustarmer Übergang von hochfrequentem Wechselstrom in die Elektroden ermöglicht, andererseits ist eine derartige Steckverbindung durch die symmetrische Gestaltung besonders bedienungssicher und bei der Verwendung federnder Kontakte sicher mechanisch fixiert.

Bei einer vorteilhaften Ausführungsform ist in dem Griffstück ein elektrischer Handschalter angeordnet, der in die Versorgungsleitungen der Nadelelektroden eingefügt ist. Ein in das Griffstück einsetzbarer Schalter weist beispielsweise einen Druck- oder Schiebekontakt auf. Damit ist es möglich, bei eingestochener Koagulationsgabel Zeitpunkt und Dauer des stromflusses mittels der das Instrument führenden Hand exakt zu kontrollieren.

Alternativ ist es auch möglich, in den Versorgungsleitungen der Nadelelektroden einen Fußschalter anzuordnen, der es ermöglicht, das Instrument ohne Zuhilfenahme der Hände ein- und auszuschalten. Die Behandlung wird dabei deutlich vereinfacht, da mit einer Hand die erfindungsgemäße Koagulationsgabel exakt führbar ist, während mit der anderen Hand beispielsweise dar Hautgewebe gestrafft wird.

Ein Ausführungsbeispiel der Erfindung wird im folgenden anhand der Zeichnungen näher erläutert. Es zeigen im einzelnen:
- Fig. 1: ein erfindungsgemäßes Instrument in einer Gesamtansicht;
- Fig. 2: den Elektrodenkopf des Instruments aus Fig. 1;
- Fig. 3: eine schematische Schnittdarstellung einer Besenreisor-Varize mit einem eingestochenen Elektrodenkopf gemaß Fig. 1 oder Fig. 2.

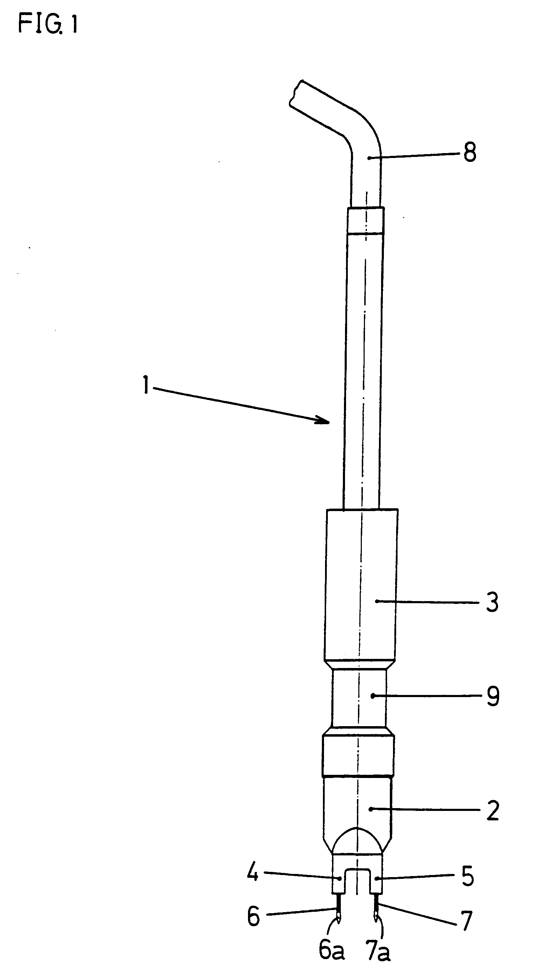

In Fig. 1 ist das erfindungsgemäße elektrochirurgische Instrument, d. h. die bipolare Koagulationsgabel, als ganzes mit dem Bezugszeichen 1 versehen. Es besteht im wesentlichen aus einem Elektrodenkopf 2, der in ein langgezogenes, zylindrisch-griffelförmiges Griffstück 3 eingesetzt ist.

In seinem vorderen Bereich ist der Elektrodenkopf 2 in etwa hosenförmig ausgebildet. Er besteht im wesentlichen aus Isolierstoff. Die Schenkel des Elektrodenkopfes 2 weisen parallel nach vorn und sind als Isolierhülsen 4 und 5 ausgebildet. In den Isolierhülsen 4 und 5 sind an deren vorderen, stumpfen Enden in Längsrichtung vorstehend Nadelelektroden 6 und 7 befestigt. Die Nadelelektroden 6 und 7 sind an dem Elektrodenkopf 2 folglich in etwa gabelartig angeordnet.

Die Nadelelektroden 6 und 7 sind in ihrem hinteren Bereich mit einer dünnen elektrischen Isolierschicht überzogen, was in der Zeichnung durch die schwarze Ausfüllung dargestellt ist. Die mit 6a und 7a bezeichneten Spitzen der Nadelelektroden 6 und 7 sind hingegen metallisch blank, d.h. unisoliert.

In das Griffstück 3 mündet von hinten ein elektrisches Kabel 8 ein, welches mit einem nicht dargestellten HF-Versorgungsteil verbunden ist. Die Leitungen des Kabels 8 verlaufen im Innern des Griffstücks 3 über einen manuellen Schalter 9, der als Ringschaltkontakt ausgeführt ist, in den Elektrodenkopf 2 und enden in den Nadelelektroden 6 und 7.

Der Elektrodenkopf 2 und das Griffstück 3 sind mechanisch und elektrisch über In dieser Darstellung nicht sichtbare Verbindungselemente miteinander lösbar verbunden.

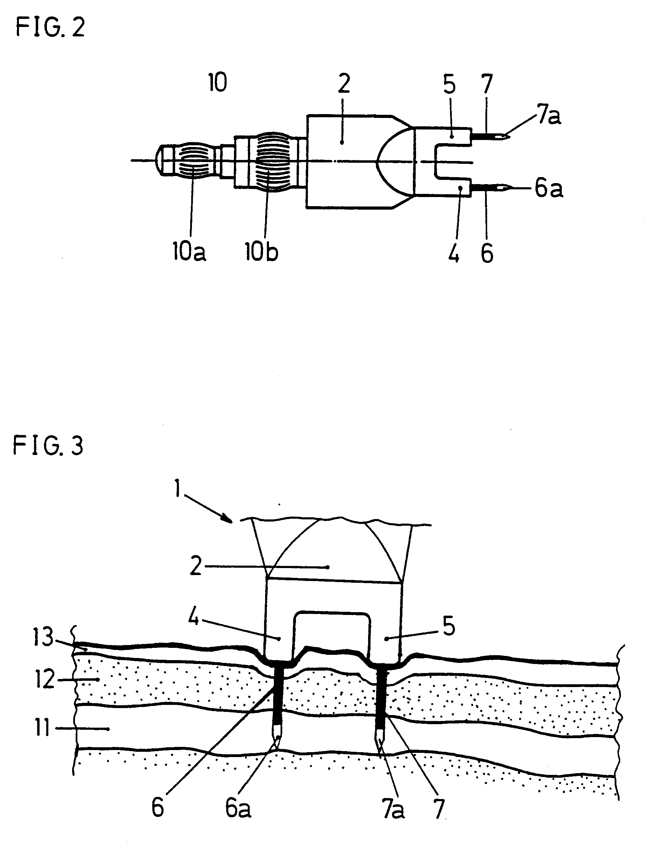

In Fig. 2 ist der Elektrodenkopf 2 aus Fig. 1 aus dem Griffstück 3 herausgenommen dargestellt. Deutlich ist der koaxiale Steckverbinder 10 erkennbar, der radial federnde Ringkontakte 10a und 10b aufweist. Diese sind mit den Nadelelektroden 6 und 7 elektrisch leitend verbunden. Das Griffstück 3 aus Fig. 3 ist mit einer in der Zeichnung nicht dargestellten Steckbuchse versehen, die den Steckverbinder 10 aufnimmt und den elektrischen Anschluß und mechanische Arretierung herstellt.

Fig. 3 zeigt eine schematische Schnittdarstellung einer typischen Behandlungssituation, bei der eine Besenreiser-Varize 11 unterhalb der Lederhaut 12 und der Oberhaut 13 eines menschlichen Körpers verläuft.

Durch die Oberhaut 13 und die Lederhaut 12 ist die Koagulationsgabel 1 mit den Nadelelektroden 6 und 7 eingestochen, und zwar derart, daß sich die Spitzen 6a und 7a sich im Querschnitt der Besenreiser-Varize 11 befinden. Dabei liegen die Isolierhülsen 4 und 5 mit ihren vorderen, stumpfen Enden als Anschlag auf der Oberhaut 13 auf.

Die Therapie mittels der erfindungsgemäßen, bipolaren Koagulationsgabel 1 ist besonders einfach und sicher durchführbar: Dazu wird zunächst ein geeigneter Elektrodenkopf 2 ausgewählt, der Nadelelektroden 6 und 7 aufweist, die hinsichtlich Länge und Abstand voneinander optimal auf den jeweiligen Einsatzzweck abgestimmt sind. Über das elektrische Kabel 8 wird die bipolare Koagulationsgabel 1 mit einem geeigneten HF-Versorungsgerät verbunden und ist damit einsatzbereit.

Daraufhin werden die Nadelelektroden 6 und 7 In eine Besenreiser-Varize 11, die unter der Hautoberfläche durch die Oberhaut 13 und die Lederhaut 12 deutlich in ihrem Verlauf erkennbar ist, eingestochen, und zwar so tief, bis die Isolierhülsen 4 und 5 des Elektrodenkopfes 2 auf der Oberhaut 13 aufsetzen. Dabei liegt die in Fig. 3 dargestellte Situation vor.

Die Nadelelektroden 6 und 7 können nicht zu tief eingestochen werden, weil die Isolierhülsen 4 und 5 einen Anschlag bilden. Durch die hosenartige Gestaltung des Elektrodenkopfes 2 hat der Operateur freie Sicht auf die zwischen den Nadelelektroden 6 und 7 verlaufende Besenreiser-Varize.

Durch Niederdrücken des manuellen Schalters 9 wird eine HF-Spannung auf die Nadelelektroden 6 und 7 gegeben. Dadurch fließt ein HF-Strom über das elektrolytisch leitende Körpergewebe entlang einer direkten Verbindungslinie zwischen den blanken Spitzen 6a und 7a der Nadelelektroden 6 und 7. Diese gedachte Verbindungslinie verläuft innerhalb der Besenreiser-Varize 11, wodurch folglich lediglich der Bereich der Besenreiser-Varize 11 einen Stromflußkanal bildet, der erhitzt wird. Durch die eng begrenzte, lokale Erwärmung wird diese in dem Abschnitt zwischen den Elektroden 6 und 7 koaguliert bzw. ausgetrocknet, d.h. verödet.

Aus der bei der Verödung der Besenreiser-Varize 11 entstehenden Erhöhung der elektrischen Gesamtimpedanz zwischen den Nadelelektroden 6 und 7 sinkt der HF-Strom, wenn die Therapie zum Erfolg geführt hat. Der Therapeut kann dies beispielsweise anhand eines Strommeßgerätes kontrollieren und den Stromfluß mittels des Schalters 9 rechtzeitig unterbrechen.

Die erfindungsgemäße bipolare Koagulationsgabel 1 ist für den Therapeuten besonders bedienungssicher, der Patient verspürt lediglich die Einstiche der dünnen und kurzen Nadelelektroden 6 und 7, was nicht besonders schmerzhaft Ist. Die Besenreiser-Varizen 11 werden gründlich und sicher verödet, wobei auf der Hautoberfläche der Oberhaut 13 keine bleibenden, kosmetisch unansehnlichen Veränderungen auftreten.

## Patentansprüche

1. Elektrochirurgisches Instrument zur Therapie von Varizen, insbesondere Besenreiser-Varizen, **dadurch gekennzeichnet,** daß das Instrument (1) zwei gabelartig angeordnete, vorstehende Nadelelektroden (6, 7) aufweist, die an unterschiedliche Pole einer elektrischen Stromquelle anschließbar sind und die jeweils teilweise mit einer dünnen elektrischen Isolierschicht umgeben sind, welche den vorderen Spitzenbereich (6a, 7a) der Nadelelektroden (6, 7) freiläßt, wobei der Übergang vom Spitzenbereich zum isolierten Bereich im wesentlichen absatzlos ist.

2. Instrument nach Anspruch 1, dadurch gekennzeichnet, daß die Nadelelektroden (6, 7) am hinteren Ende axial in Isolierhülsen (4, 5) gehaltert sind, die einen deutlich größeren Querschnitt aufweisen als die Nadelelektroden (6, 7).

3. Instrument nach Ansprüchen 1 und 2, dadurch gekennzeichnet, daß die Isolierhülsen (4, 5) die Schenkel eines in etwa hosenförmigen Elektrodenkopfes (2) bilden, an dem schenkelseitig nach vorne gerichtet die Nadelelektroden (6, 7) befestigt sind und der nach hinten mit einem griffelförmigen Griffstück (3) verbunden ist.

4. Instrument nach Anspruch 3, dadurch gekennzeichnet, daß der Elektrodenkopf (2) und das Griffstück (3) korrespondierende elektrische und mechanische Verbindungselemente (10, 10a, 10b) aufweisen, die lösbar miteinander verbindbar sind.

5. Instrument nach Anspruch 3, dadurch gekennzeichnet, daß in dem Griffstück (3) ein elektrischer Handschalter (9) angeordnet ist, der in die Versorgungsleitungen (8) der Nadelelektroden (6, 7) eingefügt ist.

6. Instrument nach einem oder mehreren der Ansprüche 1 bis 4, dadurch gekennzeichnet, daß in den Versorgungsleitungen (8) der Nadelelektroden (6, 7) ein Fußschalter angeordnet ist.

## Claims

1. An electro-surgical instrument for the treatment of varices, in particular broom-twig varices, characterised in that the instrument (1) has two projecting needle electrodes (6, 7) which are arranged fork-like and which can be connected to different poles of an electrical current source and which are each partially surrounded by a thin electrical insulating layer which leaves free the front tip region (6a, 7a) of the needle electrodes (6, 7), wherein the transition from the tip region to the insulated region is substantially stepless.

2. An instrument according to claim 1 characterised in that the needle electrodes (6, 7) are held at the rear end axially in insulating sleeves (4, 5) which are of a markedly larger cross-section than the needle electrodes (6, 7).

3. An instrument according to claims 1 and 2 characterised in that the insulating sleeves (4, 5) form the limbs of an electrode head (2) which is substantially in the shape of a pair of trousers and to which the needle electrodes (7) are secured directed forwardly at the limb end and which is connected rearwardly to a pencil-shaped handle portion (3).

4. An instrument according to claim 3 characterised in that the electrode head (2) and the handle portion (3) have corresponding electrical and mechanical connecting elements (10, 10a, 10b) which can be releasably connected together.

5. An instrument according to claim 3 characterised in that arranged in the handle portion (3) is a manual electrical switch (9) which is fitted in the supply lines (8) of the needle electrodes (6, 7).

6. An instrument according to one or more of claims 1 to 4 characterised in that a foot switch is arranged in the supply lines (8) of the needle electrodes (6, 7).

## Revendications

1. Instrument électrochirurgical pour le traitement de varices, notamment de varices Besenreiser, caractérisé en ce que l'instrument (1) présente deux électrodes à aiguilles saillantes (6, 7) disposées en forme de fourche pouvant être raccordées à des pôles différents d'une source de courant électrique et qui sont entourées respectivement partiellement par une couche d'isolation électrique mince qui laisse libre la zone de pointe avant (6a, 7a) des électrodes à aiguilles (6, 7), la transition de la zone de pointe à la zone isolée étant sensiblement sans gradin.

2. Instrument selon la revendication 1, caractérisé en ce que les électrodes à aiguille (6, 7) sont retenues à l'extrémité arrière axialement dans des douilles d'isolation (4, 5) qui ont une section transversale nettement plus grande que les électrodes à aiguille (6, 7).

3. Instrument selon les revendications 1 et 2, caractérisé en ce que les douilles d'isolation (4, 5) forment les branches d'une tête d'électrode (2) à peu près de forme bifurquée à laquelle sont fixées, côté branches, en étant orientées vers l'avant, les électrodes à aiguille (6, 7) et qui est reliée vers l'arrière à une pièce de préhension (3) en forme de crayon.

4. Instrument selon la revendication 3, caractérisé en ce que la tête d'électrode (2) et la pièce de préhension (3) présentent des éléments de liaison électriques et mécaniques correspondant (10, 10a, 10b) qui peuvent être reliés amoviblement les uns aux autres.

5. Instrument selon la revendication 3, caractérisé en ce qu'il est disposé dans la pièce de préhension (3) un interrupteur manuel électrique (9) qui est inséré dans les lignes d'alimentation (8) des électrodes à aiguille (6, 7).

6. Instrument selon l'une ou plusieurs des revendications 1 à 4, caractérisé en ce qu'il est disposé dans les lignes d'alimentation (8) des électrodes à aiguille (6, 7) un interrupteur à pédale.
